# EUROPEAN PATENT APPLICATION

(11) **EP 4 686 928 A1**
(43) Date of publication of application: **04.02.2026**
(21) Application number: 25186126.6
(22) Date of filing: 30.06.2025
(51) Int. Cl.: G01M 3/22, F17D 5/00, F17D 5/02, G01M 3/00, G01M 3/28, G01N 33/00

(54) **APPARATUS FOR ACTIVE REMOTE DETECTION OF LEAKING HYDROGEN**

(30) Priority: 29.07.2024 GB 202411048
(71) Applicant: Rolls-Royce plc, London N1 9FX (GB)
(72) Inventor: Coulson, James T J, Derby, DE24 8BJ (GB)
(74) Representative: Rolls-Royce plc

(57) **Abstract**

Apparatus for active remote detection of leaking hydrogen comprises (i) pressure tubing (102) for enclosing a length of pipework from which leaking hydrogen is to be detected; (ii) a detection vessel (112) containing a hydrogen sensor (114); and (iii) connecting tubing (106) connecting the interior of the pressure tubing to the interior of the detection vessel.

## Description

### FIELD

The invention relates to remote detection of leaking hydrogen.

### BACKGROUND

Transport of liquid or gaseous hydrogen within an apparatus is a key technical consideration in situations where hydrogen is used as a fuel. For example in transport applications (road, rail, aerospace) it is generally required to transport hydrogen from a store of liquid or gaseous hydrogen via pipework to some kind of engine (e.g. a PEM fuel cell stack or hydrogen-burning internal combustion engine) where the hydrogen is oxidised in order to generate propulsive power. Apparatus intermediate the store and the engine may be present to provide vaporisation and/or heating of a flow of hydrogen so that it is suitably conditioned prior to consumption by the engine. Transmission of hydrogen via pipework almost inevitably involves some leakage of hydrogen being conveyed therein, especially where different sections of pipework are joined together to form an interface, owing to the very small size of the hydrogen molecule. If hydrogen leaks from pipework, it can present an explosion and/or fire risk, as well as representing a loss of useful fuel. Currently, there is no apparatus for active remote detection and monitoring of leaking hydrogen or micro-flaming of leaking hydrogen around pipework or pipework interfaces. Known technology uses hydrogen sensors with limited range in high-flow environments, such as the bypass duct of a turbofan engine, or detection tape which does not provide active monitoring.

### SUMMARY

According to the invention, apparatus for active remote detection of leaking hydrogen comprises:
(i) pressure tubing for enclosing a length of pipework from which leaking hydrogen is to be detected;
(ii) a detection vessel containing a hydrogen sensor; and
(iii) connecting tubing connecting the interior of the pressure tubing to the interior of the detection vessel.

The connecting tubing may comprise a plurality of individual tubes each having a respective first end coupling the interior of the pressure tubing at a respective position thereon to the interior of the detection vessel at a first end thereof. The respective positions at which the plurality of individual tubes meet the pressure tubing may be distributed axially, azimuthally or both axially and azimuthally. The detection vessel may contain a heat sensor. The heat sensor may be a thermocouple or a thermistor. The detection vessel may comprise a dynamic pressure sensor. The apparatus may comprise a pump or pressure-drop device coupled to the detection vessel at an end thereof remote from the connecting tubing.

### BRIEF DESCRIPTION OF THE DRAWINGS

Embodiments of the invention are described below, by way of example only, with reference to the accompanying drawings, in which:
- FIG. 1: shows a portion of pipework arranged to convey liquid or gaseous hydrogen;
- FIGS. 2 & 3: show first and second portions respectively of an example apparatus which can provide active and remote detection of leaks from the pipework of FIG. 1;
- FIG. 4: shows a third portion of the example apparatus; and
- FIG. 5: shows a transverse cross-section through the pipework of FIG. 1 with the example apparatus fitted to the pipework.

### DETAILED DESCRIPTION

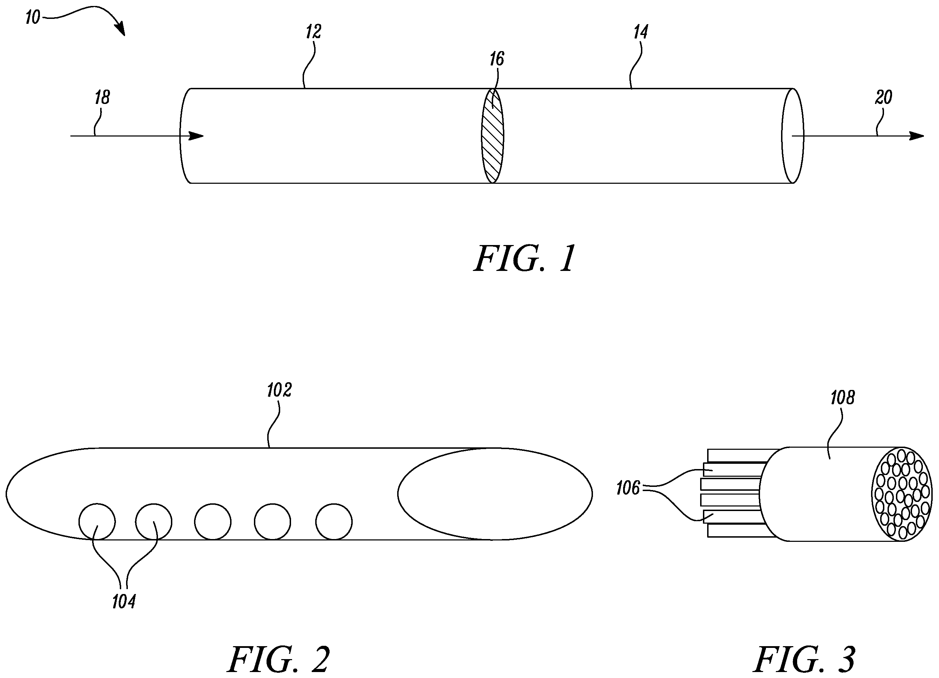

In **Figure 1****,** a portion of pipework 10 is arranged to convey either liquid or gaseous hydrogen. Hydrogen is input as indicated by arrow 18 and output as indicated by arrow 20. The pipework 10 comprises first and second sections 12, 14 which are joined end-to-end, defining an interface 16. Each of the ends of the sections 12, 14 which are joined has a respective flange (not shown) by which the sections are mechanically joined together. Gaseous hydrogen typically leaks out at the interface 16 and may also pass through the walls of the sections 12, 14, depending on the material(s) from which they are made, although at a much lower rate.

Referring to **Figure 2****,** a first portion of an example apparatus of the invention which may be applied to the pipework 10 of Figure 1 in order to actively and remotely detect leakage of gaseous hydrogen therefrom comprises pressure tubing 102 which may be fitted over the pipework 10 to enclose an axial length of the pipework 10 which includes the interface 16. A series of outlets 104 or "sniffing holes" 104 are distributed axially along the pressure tubing 102.

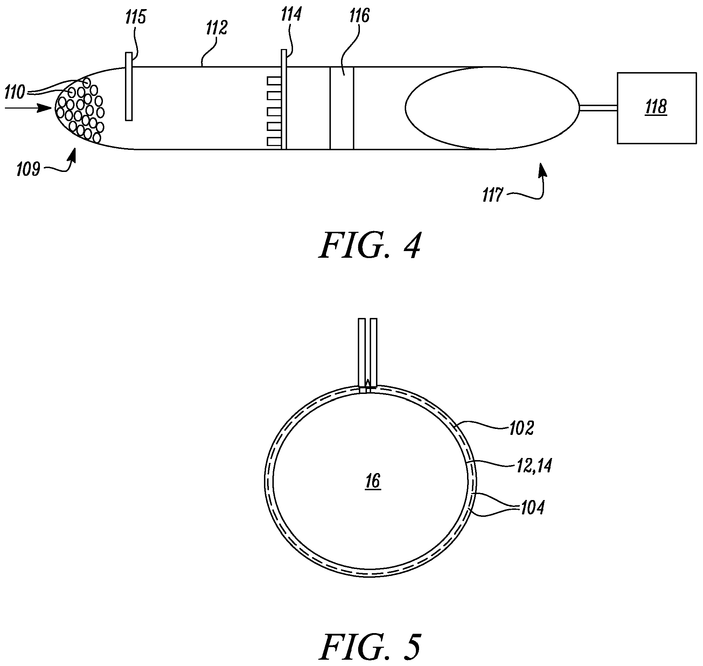

Referring also to **Figure 3****,** a second portion of the example apparatus comprises a bundle of tubes 106 each of which has a first end coupled to a respective sniffing hole 104. The bundle is kept together by means of a sleeve element 108. Referring also to **Figure 4****,** a third portion of the example apparatus comprises a detection vessel 112 having first a first end 109 provided with a plurality input holes 110. A respective second end of each tube 106 of the bundle remote from the pressure tubing 102 is coupled to a respective input hole 110. A pressure-drop device 118 is coupled to a second end 117 of the detection vessel 112 remote from the input holes 110 at the first end 109. Preferably the pressure-drop device is an ATEX-compliant pump or pressure-drop device. A hydrogen detection or hydrogen sniffing device 114 is located part-way along the detection vessel 112. Optionally, the detection vessel 112 may further include at least one of (i) a thermocouple or thermistor 115 or other heat sensor for detection of micro-flaming of hydrogen leaking from the pipework 10, and (ii) a dynamic pressure sensor 116, for line detonation detection.

In operation of the example apparatus, gaseous hydrogen leaking from the interface 16 and/or through the walls of section 12, 14 is collected by the pressure tubing 102 and enters the first end 109 of the detection vessel 112 via the bundle of tubes 106 and input holes 110 due to the action of the pressure-drop device 118. The gaseous hydrogen passes, and is detected by, the sniffing device 114 and exits the detection vessel 112 at the second end 117 thereof. If the detection vessel 112 is fitted with a heat sensor such as thermocouple or thermistor 116, then the apparatus is able additionally to detect combustion of leaking hydrogen taking place in the vicinity of the pipework 10 by detecting the heat of combustion products.

**Figure 5** shows a cross-sectional view of the pipework10 fitted with the pressure tubing 102 of the apparatus, in the plane of the interface 16. The pressure tubing 104 shown in Figure 5 has sniffing holes distributed azimuthally about a central longitudinal axis of the pipework 10. In a particular example of the apparatus, the sniffing holes 104 may be distributed axially, azimuthally, or both axially and azimuthally.

In a variant of the apparatus, the pressure-drop device 118 is omitted. The variant apparatus is thus passive and may be used where the pipework 10 is located in a pressurised volume of space, such as the bypass duct of a turbofan engine, such that pressure drop from the pressurised volume of space to atmospheric pressure is adequate to force leaking hydrogen to the second end 117 of the detection vessel 112.

Except where clearly mutually exclusive, any of the features may be employed separately or in combination with any other features and the invention extends to and includes all combinations and sub-combinations of one or more features described herein.

## Claims

1. Apparatus for active remote detection of leaking hydrogen, the apparatus comprising:
(i) pressure tubing (102) for enclosing a length of pipework from which leaking hydrogen is to be detected;
(ii) a detection vessel (112) containing a hydrogen sensor (114); and
(iii) connecting tubing (106) connecting the interior of the pressure tubing to the interior of the detection vessel.

2. Apparatus according to claim 1 wherein the connecting tubing comprises a plurality of individual tubes (106) each having a respective first end coupling the interior of the pressure tubing at a respective position thereon to the interior of the detection vessel at a first end thereof.

3. Apparatus according to claim 2 wherein the respective positions at which the plurality of individual tubes meet the pressure tubing are distributed axially, azimuthally or both axially and azimuthally.

4. Apparatus according to any preceding claim wherein the detection vessel contains a heat sensor (115).

5. Apparatus according to claim 4 wherein the heat sensor is a thermocouple or a thermistor (115).

6. Apparatus according to any preceding claim wherein the detection vessel comprises a dynamic pressure sensor (116).

7. Apparatus according to any preceding claim and comprising a pump or pressure-drop device (118) coupled to the detection vessel at an end thereof remote from the connecting tubing.
